(19) Europäisches Patentamt
European Patent Office
Office européen des brevets

(11) Publication number: **0 052 492**
**B1**

(12) # EUROPEAN PATENT SPECIFICATION

(45) Date of publication of patent specification: **29.02.84**

(51) Int. Cl.³: **C 07 C 93/06,**
A 61 K 31/135

(21) Application number: **81305387.3**

(22) Date of filing: **13.11.81**

(54) **3-Aryloxy-3-phenylpropylamines.**

(30) Priority: **14.11.80 US 206498**

(43) Date of publication of application:
**26.05.82 Bulletin 82/21**

(45) Publication of the grant of the patent:
**29.02.84 Bulletin 84/9**

(84) Designated Contracting States:
**AT BE CH DE FR GB IT LI LU NL SE**

(56) References cited:
EP - A - 0 015 418
US - A - 4 018 895

(73) Proprietor: **ELI LILLY AND COMPANY**
**307, East McCarty Street**
**Indianapolis Indiana 46285 (US)**

(72) Inventor: **Foster, Bennie Joe**
**718, Leisure Lane**
**Greenwood Indiana 46142 (US)**
Inventor: **Lavagnino, Edward Ralph**
**4010 Rommel Drive**
**Indianapolis Indiana 46208 (US)**

(74) Representative: **Crowther, Terence Roger et al,**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 052 492 B1

# 3-aryloxy-3-phenylpropylamines

This invention relates to a novel R-isomer of a 3-aryloxy-3-phenylpropylamine, which isomer has been found to be particularly effective as an antidepressant agent.

U.S. Patent Specification No. 4,018,895 discloses that 3-aryloxy-3-phenylpropylamines have antidepressant properties. Indeed, in that patent there is described the racemic form of the compound of the invention. In *Biochem Pharm, 25,* 1979—83 (1976) Wong and Bymaster discuss the activity of another racemate from the above-mentioned U.S. patent, viz. N-methyl-3-(2-methoxyphenoxy)-3-propylamine, hydrochloride. As would be expected, the (—) and (+) enantiomers of that compound are of equal activity.

However, surprisingly, the applicants have discovered that the (—) enantiomer of N-methyl-3-(2-methylphenoxy)-3-phenylpropylamine is dramatically more active than the (+)-enantiomer and, moreover, exhibits less anticholinergic side effects.

Thus, in accordance with the invention, the (—)-enantiomer of the compound of formula (I):

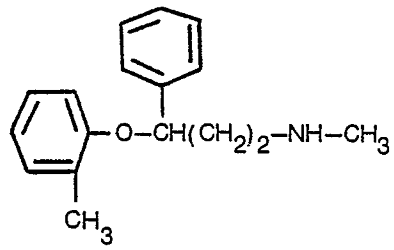

(I)

and its pharmaceutically-acceptable salts, are particularly effective antidepressant agents.

The (—) enantiomer of the invention has been shown to possess R-absolute stereochemistry.

The term "pharmaceutically-acceptable salts", as used herein, includes those acid-addition salts which are not toxic or otherwise pharmacologically unacceptable. Such salts include, for example, the hydrochloride, nitrate, phosphate, sulfate, hydrobromide, hydriodide, pyrosulfate, sulfite, metaphosphate, acetate, propionate, caprylate, formate, phthalate, citrate and lactate salts.

It is usually preferred to use the compound of formula (I) in salt form, the hydrochloride being most preferred.

Such salts can be prepared by conventional means, such as by dissolving the base in ether and adding an approximately equivalent amount of an appropriate acid.

The compound of this invention can be prepared by resolving the corresponding racemate, prepared, for example, as taught in U.S. Patent Specification No. 4,018,895.

Thus, according to the invention, there is provided a process for preparing the (—) enantiomer of formula (I), or a pharmaceutically-acceptable salt thereof, which comprises:

(a) reacting a racemic form of the compound of formula (I) with a (+)-acid so as to form a mixture of the (+) (—) and (+) (+) salts;

(b) separating the (+) (—) salt from the (+) (+) salt by fractional crystallization;

(c) reacting the (+) (—) salt so obtained with a strong base to liberate the (—) base of formula (I); and

(d) optionally salifying the free base so obtained so as to obtain a pharmaceutically-acceptable salt thereof.

The preferred (+)-acid for use in the process of the invention is L(+)-mandelic acid. The preferred strong bases are Group IA metal hydroxides, such as sodium or potassium hydroxide, or ammonum hydroxide.

The resolution should be carried out in an inert organic solvent, preferably in a nonpolar or moderately polar solvent, as discussed in detail below. It is preferred to carry out the resolution by first dissolving the racemate of formula (I) in a solvent, such as an ether, for example tetrahydrofuran, dioxane, dimethoxyethane, and diethyl ether an alcohol for example cyclohexanol, ethanol, and butanol, an alkane, for example pentane, hexane, and octane, an ester such as ethyl acetate, methyl acetate, ethyl butyrate, and methyl benzoate, a haloalkane such as dichloromethane, chloroform, and 1,2-dibromoethane or an aromatic hydrocarbon, for example benzene, toluene, ethylbenzene, and xylene. The use of ethereal solvents, especially diethyl ether, is preferred. To the solution of the racemate is added a solution or suspension of the optically-active acid in an appropriate organic solvent or suspending media such as benzene, toluene or xylene, preferably in toluene. The reaction mixture is then stirred at a temperature in the range of from about 0° to 100°C, preferably 0° to 50°C, most preferably at the ambient temperature, and the desired salt, then precipitates. The crude salt is preferably partially purified by recrystallization.

It is preferred to dissolve the crude product in a mixture of dichloromethane and ethyl acetate, and precipitate it by distilling off the dichloromethane until crystals form and then adding diethyl ether to

complete the crystallization. The mandelate salt is then dissolved in water, and converted to the desired free base by making it basic. It is preferred to isolate the product by extracting it from the aqueous solution, as with an ether, and forming the desired salt, if a salt is desired, in the ether solution.

The preferred amount of (+)-acid is 1/2 mole per mole of racemate, but amounts from about 1/2 mole to about 1 mole may be used. Use of amounts of the acid greater than 1 mole is economically unattactive.

The preferred method of preparing the racemate of formula (I) involves the dimethylation of the corresponding dimethylamino derivative of formula:

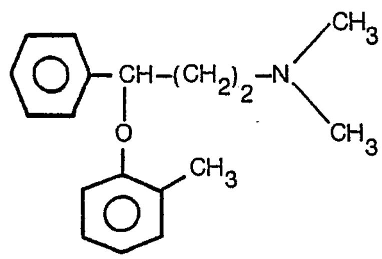

which is also described in U.S. Patent Specification No. 4,018,895. Those skilled in the art will appreciate that there are a large number of ways in which this demethylation may be accomplished. However, the applicants have found that this demethylation is advantageously effected by replacing one of the N-methyl groups with a phenoxyformyl group by reaction with phenyl chloroformate, and then hydrolyzing this group off with an aqueous base such as sodium hydroxide. This demethylation reaction may be effected at temperatures between 50 and 150°C.

The following Preparation and Examples further illustrate the preparation of the compounds of the invention.

Preparation 1

Methyl-[3-(2-methylphenoxy)-3-phenylpropyl]-carbamic acid, phenyl ester

A solution of 0.714 M (192 g) of N,N-dimethyl-$\gamma$-(2-methylphenoxy)benzenepropanamine in 500 ml. of toluene was heated to reflux, and then 0.785 M (123 g) of phenyl chloroformate was added dropwise during a 45 minute period. The addition rate of phenyl chloroformate was adjusted to maintain an even reflux rate and to moderate the bubbling of the reaction solution from generation of methyl chloride. After completion of the addition of phenyl chloroformate, the reaction mixture was refluxed for one hour and then allowed to stir at ambient temperature for 16 hours.

Next the reaction mixture was diluted with methylene chloride (1 l.) and the organic layer washed with 10% sodium hydroxide solution, saturated sodium chloride solution, 1N hydrochloric acid solution, and finally with saturated sodium chloride solution. The original basic aqueous layer was re-extracted with methylene chloride and then washed with 1N hydrochloric acid and then with saturated sodium chloride solution.

The combined organic extracts were dried by stirring over anhydrous sodium sulfate and then concentrated *in vacuo*. The title product was crystallized by trituration with hexane and then recrystallized by dissolving the crystals in diethyl ether (sparingly soluble) and methylene chloride (very soluble) and adding hexane while distilling off the more volatile solvents, thereby concentrating to a volume of approximately on liter.

The racemic product was collected and dried; weight 228 g (85% yield), m.p. 72—76°C.

Preparation 2

N-Methyl-3-(2-methylphenoxy)-3-phenylpropylamine, hydrochloride

To a solution of 0.54 M (210 g) of methyl-[3-(2-methylphenoxy)-3-phenylpropyl]carbamic acid, phenyl ester in 890 ml of propylene glycol was added (in one portion) a solution of 5.35 M (214 g) of sodium hydroxide in 890 ml of water. The reaction solution was stirred and refluxed for 20 hours (~110°C reaction temperature).

Next the reaction solution was cooled to 25°C, 2500 ml of water was added, and then the solution was extracted (3 x 400 ml) with toluene. The combined toluene extracts were dried over anhydrous magnesium sulfate and concentrated *in vacuo* to a gum.

The gummy concentrate was dissolved in 1 l. of toluene and the resulting solution was treated with gaseous hydrogen chloride until it was acidic. The acidity of the solution was checked by withdrawing an aliquot, adding water, and checking the pH of the aqueous layer.

No precipitate of the title product was seen, so the toluene solution was concentrated to dryness *in vacuo*. The viscous residue was triturated with ether until it was crystalline and then collected and washed with ether. The title product was recrystallized from methylene chloride and ethyl acetate by distilling off methylene chloride. Product weight was 135 g (85% y.); m.p. 132—135°C. TLC on a silica plate with 50:1:0.0 of chloroform:methanol: concentrated ammonium hydroxide showed a single spot. Analysis calculated for $C_{17}H_{22}NOCl$: C, 69.77; H, 7.60; N, 4.80; found C, 69.68; H, 7.34; N, 4.64.

Example 1

(—)-N-methyl-3-(2-methylphenoxy)-3-phenylpropylamine, hydrochloride

A solution of 469 g. of racemic N-methyl-3-(2-methylphenoxy)-3-phenylpropylamine, hydrochloride, in 1500 ml. of water was made basic with sodium carbonate, and was then extracted 2 times with 1 liter portions of diethyl ether. The ether extracts were combined, washed with water and dried over anhydrous magnesium sulfate. To the dry solution was added 122 g. of L(+)-mandelic acid in 2 liters of toluene, and the mixture was stirred at ambient temperature for 112 hours. The mixture was then filtered, and the solids were washed with diethyl ether to obtain 167 g. of the mandelate salt of (—)-N-methyl-3-(2-methylphenoxy)-3-phenylpropylamine. The product was recrystallized from 2 liters of a 1:1 (by volume) mixture of dichloromethane and ethyl acetate, by boiling off the dichloromethane until precipitation began and then diluting with 1 liter of diethyl ether. The resulting purified intermediate weighed 148 g.

A 748 g. portion of the mandelate salt, prepared in repeated preparations as illustrated in the paragraph above, was dissolved in 1 liter of hot methanol, and the solution was diluted with 1 liter of ambient temperature water. The resulting slurry was made basic with sodium carbonate solution, and was then extracted with two 500 ml. portions of diethyl ether, and then with two 1 liter portions of ethyl acetate. The organic layers were combined, and the mixture was washed with saturated aqueous sodium chloride and then dried over sodium sulfate and magnesium sulfate. Hydrogen chloride gas was then bubbled through the solution to precipitate the desired product as the hydrochloride salt. The solids were collected by filtration and dried to obtain 510 g. of (—)-N-methyl-3-(2-methylphenoxy)-3-phenylpropylamine, hydrochloride. The product was purified by dissolving it in 200 ml. of dichloromethane, and drying over magnesium sulfate. The dry solution was filtered, and then diluted with ethyl acetate. The mixture was heated to boil off dichloromethane, and the product crystallized and was isolated by filtration. The amount of the product was 486 g., with a melting point of 166—168°C. The optical rotation, measured as a 10 mg./ml. solution in methanol, was $\alpha_D^{25} = -38.01°$; $\alpha_{365}^{25} = -177.26°$.

Example 2

(—)-N-methyl-3-(2-methylphenoxy)-3-phenylpropylamine, hydrochloride

Eight hundred g. of racemic N-methyl-3-(2-methylphenoxy)-3-phenylpropylamine, hydrochloride, was dissolved in 2 liters of water, and was made basic with sodium carbonate. The solution was extracted twice with 2-liter portions of diethyl ether, and the ether solution was dried over anhydrous magnesium sulfate. To the solution was added a suspension of 209 g. of L(+)-mandelic acid in 200 ml. of xylene, and the mixture was stirred at ambient temperature for 22 hours. The solids were then collected and dried to obtain 372 g. of impure (—)-mandelate salt, m.p. 126—128°C. The impure salt was dissolved in 4 liters of 1:1 dichloromethane:ethyl acetate (by volume), and the mixture was boiled gently until precipitation started. A 1.5 liter portion of diethyl was then added, and the mixture was cooled and filtered to obtain 359 g. of the (—)-mandelate salt, m.p. 128—129°C. The product was recrystallized again from the same solvent system to obtain 326 g. of highly purified mandelate salt, m.p. 128—129°C.

The purified salt prepared above was dissolved in 2 liters of water, and the solution was made basic with sodium carbonate. The basic soltion was extracted twice with 1-liter portions of diethyl ether, and the organic layer were combined and dried over anhydrous sodium sulfate. Hydrogen chloride gas was then bubbled through the solution with stirring until the solution gave an acidic reaction to pH paper. The hydrochloride salt then precipitated, and was collected and recrystallized by dissolving it in 500 ml. of dichloromethane, adding 1.5 liters of ethyl acetate, and boiling the mixture gently until precipitation started, when 1 liter of diethyl ether was added. The mixture was then cooled and filtered, and the solids were dried to obtain 209 g. of (—)-N-methyl-3-(2-methylphenoxy)-3-phenylpropylamine, hydrochloride, m.p. 165—168°C. The product was recrystallized from the same solvent system to obtain 180 g. of highly purified (—)-N-methyl-3-(2-methylphenoxy)-3-phenylpropylamine, hydrochloride, m.p. 165—167°C. The optical rotation, measured as a 10 mg./ml. solution in methanol, was $\alpha_D^{25} = -37.6°$; $\alpha_{365}^{25} = -181.3°$.

Example 3

(—)-N-methyl-3-(2-methylphenoxy)-3-phenylpropylamine, hydrochloride

A 3.05 g. portion of racemic N-methyl-3-(2-methylphenoxy)-3-phenylpropylamine, hydrochloride, was dissolved in 50 ml. of water, and was made basic with sodium bicarbonate. The solution was extracted with three 50 ml. portions of diethyl ether, and the combined organic layers were washed twice with 20 ml. portions of water. The solution was dried over magnesium sulfate and concentrated under vacuum to a solid. The residue was dissolved in 100 ml. of benzene and 1.5 g. of L(+)-mandelic acid was added. The solution was then stirred for 64 hours at 23°, and was then concentrated under vacuum to a solid, to which 100 ml. of xylene was added, dissolving the residue. The solution was allowed to stand until a precipitate separated. The solids were harvested by filtration, and recrystallized from 20 ml. of xylene. The product was then suspended in water and made basic with ammonium hydroxide to prepare the free base, which was extracted with two 20 ml. portions of diethyl

ether. The organic layers were washed with two 50 ml. portions of water, dried over magnesium sulfate and treated with gaseous hydrogen chloride to prepare the hydrochloride salt, which precipitated spontaneously and was filtered from the solution. The product was recrystallized from 10 ml. of dichloromethane and 30 ml. of ethyl acetate by the addition of 50 ml. of diethyl ether. Ninety mg. of the desired product, m.p. 156—157°, was obtained. The optical rotation, measured as described above, was $\alpha_D^{25} = -29.3°$; $\alpha_{365}^{25} = -146.02°$.

Both in vivo and in vitro test precedures have been used to establish the efficacy and safety of the compound of this invention tested as the hydrochloride salt. It will be understood by the skilled reader that the art has developed a group of biochemical tests which are used to predict antidepressant activity. The reader is directed to two articles, Adolphe et al., The Neuropharmacology of Depression, *Diseases of the Nervous System,* 841—46 (1877), and Spencer, Review of the Pharmacology of Existing Antidepressants, *Br. J. Clin. Pharmac. 4,* 57S—68S (1977). These articles clearly explain the mechanism of depression, which occurs when the nervous system becomes unable to pass impulses between neurons in adequate numbers at adequate speeds. One of the major causes of depression has been found to be a condition wherein inadequate monoamines are present in the central nervous system. The most important of these amines are norepinephrine, serotonin and 3,4-dihydroxyphenylethylamine (dopamine). A class of drugs called the monoamine uptake inhibitors have been found to be effective in ameliorating this condition, apparently by inhibiting the reuptake of the amines by the synapses, the nerve terminals from which the amines were released.

The compound of this invention belongs to the class of monoamine uptake inhibitors, as does the racemate from which it is isolated, according to U.S. Patent 4,018,895. The tests reported below report the remarkable efficacy of the compound of this invention as a monoamine uptake inhibitor, compared to the racemate from which it is isolated and the corresponding (+)-enantiomer.

Monoamine uptake inhibition was measured by the in vitro method of Wong and Bymaster, cited above. In this method, male Sprague-Dawley rats weighing 110—150 g. were killed by decapitation, and the whole brains were removed and dissected. Crude preparations of synaptosomes were prepared from 10% homogenates of specific areas of the brain in 0.32 M sucrose and 10 mM glucose by differential centrifugation, according to the method of Gray and Whittaker, *J. Anat. 96,* 79 (1962).

Uptake of [$^3$H]monoamines by synaptosomes was determined as follows. A portion of a synaptosome preparation equivalent to 1 mg. of protein was incubated at 37°. for 5 minutes in 1 or 3 ml. of Krebs bicarbonate medium containing an additional 10mM of glucose, 0.1 mM iproniazid, 1 mM ascorbic acid, 0.17 mM ethylenediaminetetraacetic acid and [$^3$H]monoamine at a specified concentration. The incubation mixtures were immediately diluted with 2 ml. of ice-chilled Krebs bicarbonate buffer containing 1 mM non-radioactive monoamine. Synaptosomes were harvested by centrifugation, and rinsed with 5 ml. of cold buffer into counting vials containing 10 ml. of scintillaton fluid. Radioactivity was measured with a liquid scintillation spectrometer, and the amount of [$^3$H]-monoamine accumulated by the synaptosomes at 4° represented the background count and was subtracted from the results obtained on all samples.

Synaptosomes isolated from the hypothalamus were found to accumulate [$^3$H]norepinephrine at $3.9 \times 10^{-12}$ moles/mg. protein, when tested without any drug present in control experiments. Addition of the compound of this invention to the synaptosome preparation reduced the uptake of monoamine as shown in the table below.

| Concentration | Uptake |
|---|---|
| $1 \times 10^{-9}$M | $3.1 \times 10^{-12}$moles/mg. protein |
| 2 | 2.2 |
| 3 | 3.1 |
| 10 | 1.4 |
| 30 | 1.0 |
| 50 | 0.9 |
| 100 | 0.8 |

The above data were plotted on a log scale to determine the concentration of the compound which inhibited half of the uptake activity. The value ($IC_{50}$ value) was $4 \times 10^{-9}$M.

The same test was carried out on the (+)-enantiomer of the compound of this invention, and upon the racemic mixture. The $IC_{50}$ value for the (+)-enantiomer was $40 \times 10^{-9}$M, and the value for the racemic mixture was $9 \times 10^{-9}$M. Thus, the potency of the compound of this invention in inhibiting the

uptake of norepinephrine is about 10 times as great as is the activity of the corresponding (+)-enantiomer.

The effect of the compound of this invention on the uptake of [$^3$H]serotonin and [$^3$H]dopamine was also determined, by the same test method, using synaptosomes isolated from the corpus striatum for the determination of dopamine uptake and synaptosomes of cerebral cortex for serotonin uptake. It was found that the compound of this invention inhibits the uptake of norepinephrine much more effectively than it inhibits the uptake of the other two monoamines. The $IC_{50}$ values for the uptake of dopamine and serotonin were 4 and 1 $\times$ $10^{-6}$M, respectively.

Thus, the compound of this invention is selective in its effect, in that it inhibits norepinephrine uptake much more efficiently than it inhibits the uptake of other monoamines. Many effective antidepressant drugs also inhibit the uptake of norepinephrine most efficiently.

The compound of this invention has also been tested in live animals to determine its ability to inhibit the uptake of norpinephrine. The test animals were male Sprague-Dawley rats weighing 110—150 g., which were treated in groups of 5 with the test compound at the doses and times specified, and were killed by decapitation. The hypothalamus was removed from each brain, and homogenized in a medium as described in the in vitro test above. Aliquots of the homogenate containing 1 mg. of protein were used; the incubation times, medium and temperature were as described in the test above.

When the compound of this invention was administered 1 hour before sacrifice at rates of 1, 3 and 10 mg./kg., intraperitoneally, the uptake of [$^3$H]-norephinephrine was reduced by 35, 58 and 68%, respectively, from the control value. The $ED_{50}$ value was calculated to be 2.1 mg./kg., averaging three independent determinations. The $ED_{50}$ values were also determined for the (+)-enantiomer and the racemate, and were found to be 6.3 and 3.4 mg./kg. respectively.

The data were expressed another way by plotting the blood level of the compound against the rate of [$^3$H]norepinephrine uptake. The plot showed that the $ED_{50}$ concentration in blood plasma ws about 14 $\times$ $10^{-9}$ g./ml. for the compound of this invention. The $ED_{50}$ on the same basis for the (+)-enantiomer was 110 $\times$ $10^{-9}$ g./ml., nearly eight times as much.

The duration of inhibition of norepinephrine uptake by the compound of this invention, was observed, by sacrificing animals which received 10 mg./kg. of the compound at times up to 4 hours after administration of the compound. It was found that the maximum inhibition of norepinephrine uptake in this experiment was more than 80%, and remained approximately constant for 2 hours. Four hours after administration, about 65% inhibition remained. In contrast, the (+)-enantiomer gave a maximum inhibition of 70%, which had fallen to 50% and 35% at the second and fourth hours.

The neurotoxic agent 6-hydroxydopamine has the property of destroying norepinephrinergic terminals of the central and peripheral nervous systems. It was found that, 3 days after injection of a male Sprague-Dawley rat with 6-hydroxydopamine, intraventricularly, the uptake of norepinephrine by synaptosomes of the hypothalamus decreased from a rate of 5.6 $\times$ $10^{-12}$ to 2.2 $\times$ $10^{-12}$ moles/mg. of protein. When the compound of this invention was administered intraperitoneally before the injection of 6-hydroxydopamine, the rates of norepinephrine uptake were not as severely reduced. Doses of 1, 3 and 6 mg./kg. reduced the inhibition of uptake by 15, 43 and 70%, respectively. The $ED_{50}$ was calculated to be 4 mg./kg. The $ED_{50}$ of the (+)-enantiomer was also evaluated in the same way, and found to be about 22 mg./kg.

The compound of this invention has been evaluated in an in vitro test system to determine its anticholinergic activity.

Based on the results of this investigation, the anticholinergic activity of the compound of this invention is significantly less than that of the corresponding (+)-enantiomer or the racemate. Anticholinergic activity is a harmful side effect of many drugs. The test was carried out on 2 mm. $\times$ 30 mm. strips of fresh guinea pig trachea. The stips were suspended in 10 ml. organ baths containing physiological salt solution at 37.5°, and aerated with a mixture of 5% carbon dioxide and 95% oxygen. The tracheal strips were attached to isometric transducers connected to an automatic recorder, and were allowed to equilibrate under a tension of 2 g. for at least 2 hours before addition of drug. Dose-response curves were obtained by adding increasing concentrations of drug to the bath, in approximately 3-fold increments. The concentration of drug was increased only after the previous concentration had produced its maximum response and remained constant. Consecutive dose-response curves on a given tissue were always separated by 1 hour of washing with fresh physiological salt solution to assure maximum washout of drug.

Dissociation constants were determined using acetylcholine as the agonist. In this method, the ratio of concentrations of acetylchlorine giving equal responses in the presence and in the absence of the compound of this invention was determined at varous concentrations of the compound. The logarithm of (dose ratio—1) plotted against the negative logarithm of the molar concentration of compound yielded a straight line, the slope of which was always close to the theoretical value of unity. The method of calculation is clearly discussed by Arunlakshana and Schild, Some Quantitative Uses of Drug Antagonists, *Brit. J. Pharmacol. 14* 48—58 (1959).

From the plots, the negative logarithm of the dissociation constant of the compound of this invention was found to be 5.26, and the corresponding values for the (+)-enantiomer and the racemate

to be 6.04 and 5.72, respectively. Accordingly, anticholinergic activity of the compound of this invention is only 1/6th as great as the activity of the (+)-enantiomer, and only 1/3 as great as that of the racemate. The likelihood for anticholinergic side effects of the compound of this invention is accordingly much less than that of the corresponding (+)-enantiomer or racemate.

The apomorphine hypothermia antagonism test is used to evaluate antidepressant drugs, since it has been found that antidepressants antagonize the reduction in body temperature caused by apomorphine. The test was carried out as follows. Mice were injected with a dose of apomorphine which will reduce body temperature (measured rectally) by about 4°. The compound of this invention was injected intraperitoneally 30 minutes before the injection of the dose of apomorphine, and the body temperature was measured 30 minutes after injection of the apomorphine. The compound of this invention gave 50% antagonism of the hypothermia at a dose of 0.16 mg./kg. It was accordingly much more active than the corresponding (+)-enantiomer, which gave 50% antagonism only at 2.26 mg./kg.

A similar test was carried out by administering the apomorphine intraperitoneally and the compound of this invention orally. It was found that the highest antagonism produced by the compound of this invention at 1 mg./kg. was 35%; the corresponding (+)-enantiomer gave 50%, but only at 3 mg./kg.

Another test applied to the compound was one designed to detect electrophysiological effects in canine cardiac conducting tissue, sometimes called Purkinje fibers. It was found that the compound of this invention suppressed the rate of rise and duration of the action potential at $8 \times 10^{-6}$M concentration, whereas the (+)enantiomer was effective only at $37 \times 10^{-6}$, and the racemate was equally effective only at $18 \times 10^{-6}$M.

Acute toxicity tests have been carried out by injecting the compound of this invention at large doses in mice. It was found that no deaths occurred at doses below 90 mg./kg., with either the compound of this invention or its (+)-enantiomer. At lower doses, the compound caused obvious effects on the central nervous system, which effects disappeared quickly, within 40—60 minutes. The effects caused by comparable doses of the (+)-enantiomer were similar, but lasted much longer. For example, the mice treated with the (+)-enantiomer at 60 mg./kg. were still stimulated 5 hours after administration.

The compound of this invention is used as an antidepressant by administering to a human suffering from depression an effective antidepressant dose of the compound. Effective antidepressant doses are found in the range from about 0.1 mg.kg. to about 25 mg./kg., or from about 6 mg./day to about 1500 mg./day as a total dose for a human. The preferred dose range is from about 0.5 mg./kg. to about 5 mg./kg., or from about 30 mg./day to about 300 mg./day for a human. The compound may be administered two or more times per day, by dividing the dose appropriately for each administration, or may be administered only once. The route of administration of the compound is not a critical matter. The compound is orally absorbed, it has been found, and oral administration is usually preferred, because of its convenience. However, the compound may be effectively administered subcutaneously, by injection, or, in particular cases, by other routes of administration such as sub-lingual discs or suppositories.

Pharmaceutical compositions with which to administer the compound may be formulated as is usual in the pharmaceutical chemistry art, and may be of any of the commonly used types. When oral administration is to be used, it is usually most convenient and economical to prepare a tablet or a capsule of the compound; capsules of the compound are prepared in the usual manner, simply by diluting the compound with a pharmaceutically acceptable carrier, and filling the desired quantity into a convenient sized capsule.

Tablets, as is well known to pharmaceutical chemists, are somewhat harder to prepare, but can be produced both by direct compression and granulation-compression. As the examples below illustrate, the usual types of diluents, excipients, lubricants and binding agents may be used in preparing tablets of the compound of this invention.

When liquid dosage forms of the compound are to be prepared, it is obviously advantageous to have the compound in the form of a water-soluble salt. Such salts may be dissolved in water for injection, or dissolved in water, appropriately flavored and admixed with pharmaceutically acceptable preservatives, as an orally administered elixir.

Formulations of the compound adapted for oral administration are preferred, and tablets and capsules are the preferred formulations. Formulations supplying from about 5 mg. to about 500 mg. per dosage unit are particularly useful; dosage units supplying from about 10 to about 100 mg. per unit are preferred.

The following are typical formulations. In all cases, the compound is in the hydrochloride salt form.

Capsules

For 5 mg. per capsule:

| | |
|---|---|
| Compound | 5 mg. |
| Pregelatinized starch NF | 295 mg. |

The compound and starch are thoroughly mixed and filled into hard gelatin capsules.

For 25 mg. per capsule:

| | |
|---|---|
| Compound | 25 mg. |
| Microcrystalline cellulose NF | 123.4 mg. |
| Pregelatinized starch NF | 36.1 mg |
| Silicone fluid | 1.9 mg. |

The silicone fluid is thoroughly mixed with a portion of the excipients. The compound and remainder of the excipients are added and all ingredients thoroughly mixed together. The mix is filled into hard gelatin capsules.

For 50 mg. per capsule:

| | |
|---|---|
| Compound | 50 mg. |
| Pregelatinized starch NF | 168.4 mg. |
| Starch NF | 82.9 mg. |
| Silicone fluid | 1.6 mg. |

Processing instructions are the same as for the 25 mg. capsule.

For 500 mg. per capsule:

| | |
|---|---|
| Compound | 500 mg. |
| Pregelatinized starch NF | 400 mg. |

The compound and starch are thoroughly mixed and filled into hard gelatin capsules.

Tablets

Direct compression—for 10 mg. per tablet

| | |
|---|---|
| Compound | 10 mg. |
| Microcrystalline cellulose NF | 233.7 mg. |
| Starch NF | 45.0 mg. |
| Stearic acid NF | 6.0 mg. |
| Magnesium stearate NF | 3.0 mg. |
| Colloidal silicon dioxide NF | 0.9 mg. |

The drug and other ingredients are thoroughly mixed and compressed into tablets.

Slugged Formula—for 25 mg. per tablet

| | |
|---|---|
| Compound | 25 mg. |
| Microcrystalline cellulose NF | 249.7 mg. |
| Lactose USP | 25.0 mg. |
| Pregelatinized starch NF | 10.0 mg. |
| Stearic acid NF | 8.0 mg. |
| Magnesium stearate NF | 3.3 mg. |
| Colloidal silicon dioxide NF | 2.4 mg. |

The compound, lactose USP, pregelatinized starch NF, 30% of the microcrystalline cellulose NF, 80% of the stearic acid NF, and 50% of the colloidal silicon dioxide NF are thoroughly mixed and compressed into slugs. The slugs are sieved to form granules. The granules, magnesium stearate NF, and the remaining microcrystalline cellulose NF, stearic acid NF, and colloidal silicon dioxide NF are thoroughly mixed and compressed into tablets.

Wet granulation—for 50 mg. per tablet:

| | |
|---|---|
| Compound | 50 mg. |
| Dibasic calcium phosphate USP | 112.9 mg. |
| Lactose USP | 105.0 mg. |
| Microcrystalline cellulose NF | 60.0 mg. |
| Starch NF | 9.0 mg. |
| Stearic acid NF | 4.5 mg. |
| Magnesium stearate NF | 1.5 mg. |

The compound, dibasic calcium phosphate USP, and lactose USP are thoroughly mixed, then wet massed with a 1:1 mixture of denatured alcohol and purified water USP. The wet mass is sized, dried, and the dry mass is sieved to form granules. The granulation, microcrystalline cellulose NF, starch NF, stearic acid NF, and magnesium stearate NF, are thoroughly mixed and compressed into tablets.

**Claims for the Contracting States: BE CH DE GB IT LI LU NL SE**

1. The (—)-enantiomer of the compound of formula (I):

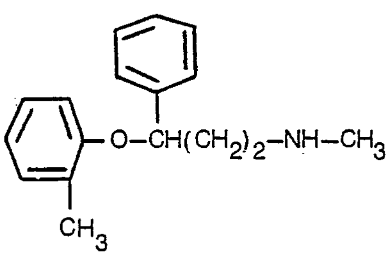

(I)

or a pharmaceutically-acceptable salt thereof.

2. (—)-N-Methyl-3-(2-methylphenoxy)-3-phenylpropylamine.

3. (—)-N-Methyl-3-(2-methylphenoxy)-3-phenylpropylamine, hydrochloride.

4. A pharmaceutical formulation comprising as an active ingredient the (—) enantiomer of formula (I), or a pharmaceutically-acceptable salt thereof, as claimed in any one of claims 1 to 3, associated with at least one pharmaceutically-acceptable carrier therefor.

5. The (—) enantiomer of formula (I), or a pharmaceutically-acceptable salt thereof, as claimed in any one of claims 1 to 3 for use as an antidepressant.

6. A process for preparing the (—) enantiomer of formula (I), or a pharmaceutically-acceptable salt thereof, as claimed in any one of claims 1 to 3 which comprises:

(a) reacting a racemic form of the compound of formula (I) with a (+)-acid so as to form a mixture of the (+) (—) and (+) (+) salts;

(b) separating the (+) (—) salt from the (+) (+) salt by fractional crystallization;

(c) reacting the (+) (—) salt so obtained with a strong base to liberate the (—) base of formula (I); and

(d) optionally salifying the free base so obtained so as to obtain a pharmaceutically-acceptable salt thereof.

**Claims for the Contracting State: AT**

1. A process for preparing the (—)-enantiomer of the compound of formula (I):

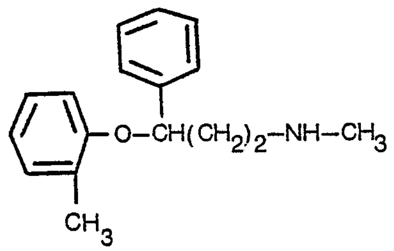

(I)

or a pharmaceutically-acceptable salt thereof, which comprises:

(a) reacting a racemic form of the compound of formula (I) with a (+)-acid so as to form a mixture of the (+) (—) and (+) (+) salts;

(b) separating the (+) (—) salt from the (+) (+) salt by fractional crystallization;

(c) reacting the (+) (—) salt so obtained with a strong base to liberate the (—) base of formula (I); and

(d) optionally salifying the free base so obtained so as to obtain a pharmaceutically-acceptable salt thereof.

**Revendications pour les Etats contractants BE CH DE FR GB IT LI LU NL SE**

1. Enantiomère (—) du composé de formule (I):

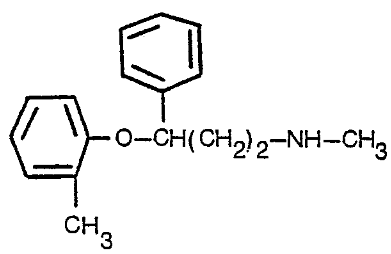

(I)

ou un de ses sels pharmaceutiquement acceptables.

2. La (—)-N-méthyl-3-(2-méthylphénoxy)-3-phénylpropylamine.

3. Le chlorhydrate de (—)-N-méthyl-3-(2-méthylphénoxy)-3-phénylpropylamine.

4. Formulation pharmaceutique comprenant, comme ingrédient actif, l'énantiomère (—) de formule (I) ou un de ses sels pharmaceutiquement acceptables suivant l'une quelconque des revendications 1 à 3, en association avec au moins un support pharmaceutiquement acceptable pour cet énantiomère ou ce sel.

5. Enantiomère (—) de formule (I) ou un de ses sels pharmaceutiquement acceptables suivant l'une quelconque des revendications 1 à 3 en vue de l'utiliser comme antidépresseur.

6. Procédé de préparation de l'énantiomére (—) de formule (I) ou d'un ses sels pharmaceutiquement acceptables suivant l'une quelconque des revendications 1 à 3, caractérisé en ce qu'il comprend les étapes qui consistent à:

(a) faire réagir une forme racémique du composé de formule (I) avec un acide (+) afin de former un mélange des sels (+) (—) et (+) (+);

(b) séparer le sel (+) (—) du sel (+) (+) par cristallisation fractionnée;

(c) faire réagir le sel (+) (—) ainsi obtenu avec une base forte pour libérer la base (—) de formule (I); et

(d) éventuellement salifier la base libre ainsi formée pour obtenir un de ses sels pharmaceutiquement acceptables.

**Revendication pour l'Etat contractant: AT**

Procédé de préparation de l'énantiomére (—) de formule (I)

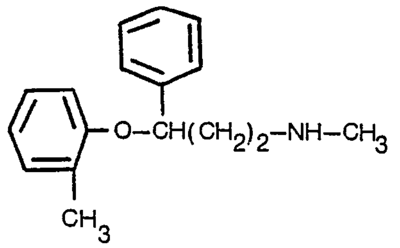

(I)

ou d'un ses sels pharmaceutiquement acceptables suivant l'une quelconque des revendications 1 à 3, caractérisé en ce qu'il comprend les étapes qui consistent à:

(a) faire réagir une forme racémique du composé de formule (I) avec un acide (+) afin de former un mélange des sels (+) (—) et (+) (+);

(b) séparer le sel (+) (—) du sel (+) (+) par cristallisation fractionnée;

(c) faire réagir le sel (+) (—) ainsi obtenu avec une base forte pour libérer la base (—) de formule (I); et

(d) éventuellement salifier la base libre ainsi formée pour obtenir un de ses sels pharmaceutiquement acceptables.

**Patentansprüche für die Vertragsstaaten BE CH DE FR GB IT LI LU NL und SE**

1. (—)-Enantiomeres der Verbindung der Formel (I)

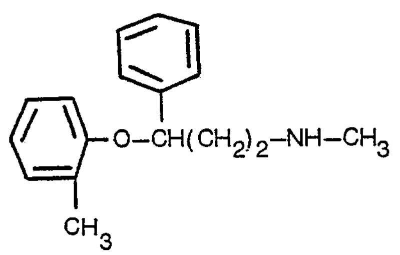

(I)

oder ein pharmazeutisch annehmbares Salz hiervon.

2. (—)-N-Methyl-3-(2-methylphenoxy)-3-phenylpropylamin.

3. (—)-N-Methyl-3-(2-methylphenoxy)-3-phenylpropylaminhydrochlorid.

4. Pharmazeutische Formulierung, dadurch gekennzeichnet, daß sie das (—)-Enantiomere der Formel (I) oder ein pharmazeutisch annehmbares Salz hiervon gemäß einem der Ansprüche 1 bis 3 als Wirkstoff in Verbindung mit wenigstens einem pharmazeutisch Annehmbaren Träger hierfür enthält.

5. Verwendung des (—)-Enantiomeren der Formel (I) oder eines pharmazeutisch annehmbaren Salzes hiervon gemäß einem der Ansprüche 1 bis 3 als Antidepressivum.

6. Verfahren zur Herstellung des (—) Enantiomeren der Formel (I) oder eines pharmazeutisch annehmbaren Salzes hiervon gemäß einem der Ansprüche 1 bis 3, dadurch gekennzeichnet, daß man

(a) eine racemische Form der Verbindung der Formel (I) mit einer (+)-Säure umsetzt und so ein Gemisch aus dem (+) (—)-Salz und dem (+) (+)-Salz bildet,

(b) das (+) (—)-Salz von dem (+) (+)-Salz durch fraktionierte Kristallisation abtrennt,

(c) das so erhaltene (+) (—)-Salz mit einer starken Base zur Reaktion bringt und so die (—)-Base der Formel (I) freisetzt und

(d) die erhaltene freie Base Gewünschtenfalls in ein Salz überführt und so zu einem pharmazeutisch annehmbaren Salz hiervon gelangt.

**Patentanspruch für den Vertragsstaat AT**

Verfahren zur Herstellung des (—)-Enantiomeren der Verbindung der Formel (I)

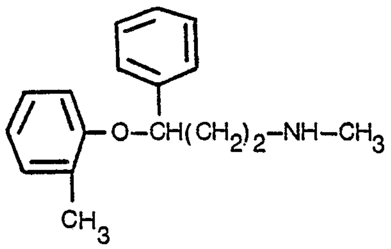

(I)

oder eines pharmazeutisch annehmbaren Salzes hiervon, dadurch gekennzeichnet, daß man

(a) eine racemische Form der Verbindung der Formel (I) mit einer (+)-Säure umsetzt und so ein Gemisch aus dem (+) (—)-Salz un dem (+) (+)-Salz bildet,

(b) das (+) (—)-Salz von dem (+) (+)-Salz durch fraktionierte Kristallisation abtrennt,

(c) das so erhaltene (+) (—)-Salz mit einer starken Base zur Reaktion bringt und so die (—)-Base der Formel (I) freisetzt und

(d) die erhaltene freie Base Gewünschtenfalls in ein Salz überführt und so zu einem pharmazeutisch annehmbaren Salz hiervon gelangt.